# EUROPEAN PATENT SPECIFICATION

(11) **EP 2 086 630 B1**
(45) Date of publication and mention of the grant of the patent: **03.11.2010**
(21) Application number: 07709829.1
(22) Date of filing: 25.01.2007
(51) Int. Cl.: A61N 1/05

(54) **IMPLANTABLE MEDICAL ELONGATED MEMBER INCLUDING EXPANDABLE FIXATION MEMBERS**
IMPLANTIERBARES MEDIZINISCHES LÄNGLICHES ELEMENT MIT EXPANDIERBAREN BEFESTIGUNGSELEMENTEN
ÉLÉMENT MÉDICAL IMPLANTABLE ALLONGÉ COMPRENANT DES FIXATIONS EXTENSIBLES

(30) Priority: 31.10.2006 US 591174
(43) Date of publication of application: 12.08.2009
(73) Proprietor: MEDTRONIC, INC., Minneapolis, Minnesota 55432 (US)
(72) Inventor: GERBER, Martin, T., Maple Grove, MN 55369 (US)
(74) Representative: Hughes, Andrea Michelle
(86) International application number: PCT/US2007/001965
(87) International publication number: WO 2008/054446

(56) References cited:
- US-A1- 2002 072 787
- US-A1- 2002 156 383
- US-A1- 2004 034 401
- US-A1- 2004 230 282
- US-A1- 2006 036 307
- US-A1- 2006 095 078

## Description

### TECHNICAL FIELD

The invention relates to medical device systems, and more particularly, to elongated members in medical device systems.

### BACKGROUND

Electrical stimulation systems may be used to deliver electrical stimulation therapy to patients to treat a variety of symptoms or conditions such as chronic pain, tremor, Parkinson's disease, multiple sclerosis, spinal cord injury, cerebral palsy, amyotrophic lateral sclerosis, dystonia, torticollis, epilepsy, pelvic floor disorders, gastroparesis, muscle stimulation (e.g., functional electrical stimulation (FES) of muscles) or obesity. An electrical stimulation system typically includes one or more neurostimulation leads coupled to a neurostimulator.

The neurostimulation lead may be percutaneously or surgically implanted in a patient on a temporary or permanent basis such that at least one stimulation electrode is positioned proximate to a target stimulation site. The target stimulation site may be, for example, a nerve or other tissue site, such as a spinal cord, pelvic nerve, pudendal nerve, stomach, bladder, or within a brain or other organ of a patient, or within a muscle or muscle group of a patient. The one or more electrodes located proximate to the target stimulation site may deliver electrical stimulation therapy to the target stimulation site in the form of electrical pulses.

Electrical stimulation of a sacral nerve may eliminate or alleviate some pelvic floor disorders by influencing the behavior of the relevant structures, such as the bladder, sphincter and pelvic floor muscles. Pelvic floor disorders include urinary incontinence, urinary urge/frequency, urinary retention, pelvic pain, bowel dysfunction, and male and female sexual dysfunction. The organs involved in bladder, bowel, and sexual function receive much of their control via the second, third, and fourth sacral nerves, commonly referred to as S2, S3 and S4 respectively. Thus, in order to deliver electrical stimulation to at least one of the S2, S3, or S4 sacral nerves, a neurostimulation lead is implanted proximate to the sacral nerve(s).

Electrical stimulation of a peripheral nerve, such as stimulation of an occipital nerve, may be used to induce paresthesia. Occipital nerves, such as a lesser occipital nerve, greater occipital nerve or third occipital nerve, exit the spinal cord at the cervical region, extend upward and toward the sides of the head, and pass through muscle and fascia to the scalp.
Pain caused by an occipital nerve, e.g. occipital neuralgia, may be treated by implanting a lead proximate to the occipital nerve to deliver stimulation therapy.

In many electrical stimulation applications, it is desirable for a stimulation lead to resist migration following implantation. For example, it may be desirable for the electrodes disposed at a distal end of the implantable medical lead to remain proximate to a target stimulation site in order to provide adequate and reliable stimulation of the target stimulation site. In some applications, it may also be desirable for the electrodes to remain substantially fixed in order to maintain a minimum distance between the electrode and a nerve in order to help prevent inflammation to the nerve and in some cases, unintended nerve damage. Securing the stimulation lead at the target stimulation site may minimize lead migration.
US 2004/034401 discloses a therapy delivery device having adjustable fixation members that are retractable in recesses.
US 2006/095078 discloses a therapy delivery device with fixation elements arranged between electrodes.
US 2004/0230282 discloses a therapy delivery device with an electrode arranged, between hydrogel fixation members. A delivery sheath is used to compress the fixation members during implantation.

### SUMMARY

The invention provides an apparatus as defined in claim 1.

The therapy may be electrical stimulation, drug delivery, or both. In one embodiment, the elongated member is an implantable medical lead that is coupled to an external or implantable electrical stimulator, which is configured to deliver electrical stimulation therapy to a target stimulation site in a patient via the lead, and more specifically, via at least one electrode disposed adjacent to a distal end of a lead body of the lead. In another embodiment, the elongated member is a catheter configured to deliver a fluid, such as pharmaceutical agents, insulin, pain relieving agents, gene therapy agents, or the like from an external or implantable fluid reservoir and/or pump to a target tissue site in a patient.

The expandable fixation member/may be, for example, hydrogel fixation member or a shape memory fixation members. Prior to implantation in a patient, the expandable fixation members are in a first state and have a first dimension. The elongated member has a relatively small profile when the expandable fixation members are in the first state because the expandable fixation members are in a generally unexpanded state and are at least partially disposed in recesses defined by the elongated member, which minimize or eliminate the amount the expandable fixation members protrude past an outer surface of the elongated member. Upon implantation in a patient, the expandable fixation members expand to a second state and extend past the outer surface of the elongated member to engage with surrounding tissue. In the second state, the expandable fixation members have a second dimension, which is greater than the first dimension, thereby enlarging the profile of at least a portion of the elongated member. By engaging with surrounding tissue, the expandable fixation members help substantially fix a position of the elongated member to (e.g., at or near) the target therapy delivery site, thereby reducing migration of the elongated member. For example, if the elongated member is a lead, the expandable fixation members help substantially fix a position of electrodes of the lead proximate to (e.g., at or near) the target stimulation site, thereby reducing lead migration.

In the second state, the expandable fixation members may define protrusions of any suitable shape and size that are capable of engaging with surrounding tissue when implanted in a patient. For example, the expandable fixation members may define tines or flange-like structures when expanded. The expandable fixation members may be disposed at any suitable location along the lead body. Examples of suitable locations include a position between a proximal end of the lead body and one or more electrodes of the lead, between the distal end of the lead body and the one or more electrodes, or between the distal end or one of the electrodes and another expandable fixation member. According to the invention, the expandable fixation members are be disposed both proximally and distally to the one or more electrodes and preferably between electrodes.

There is also diclosed a method for implanting an elongated member in a patient. The method comprises advancing the elongated member through the introducer to a target therapy delivery site to deploy the expandable fixation member into tissue of the patient proximate to the target therapy delivery site. Upon implantation in the patient, the expandable fixation member expands and extends from the elongated member to engage with surrounding tissue.

### BRIEF DESCRIPTION OF DRAWINGS

FIG 1A is a schematic perspective view of a therapy system, which includes an electrical stimulator coupled to an implantable medical lead.

FIG. 1B illustrates the implantation of a medical lead at a location proximate to an occipital nerve

FIG. 2 is a block diagram illustrating various components of the electrical stimulator and implantable lead of the therapy system of FIG. 1.

FIG. 3 is a perspective view of the implantable medical lead of FIGS. 1A, 1B, and 2 and illustrates hydrogel fixation members extending from a lead body to engage with surround tissue.

FIG. 4A is a schematic cross-sectional view of the lead of FIG. 3 taken along line 4-4 in FIG. 3.

FIG. 4B is a schematic cross-sectional view of the lead of FIG. 3 taken along line 4-4 in FIG. 3 and including an expandable sleeve disposed around the lead body to retain the hydrogel fixation members within their respective recesses.

FIGS. 5-9 illustrate alternate arrangements of hydrogel fixation members on a lead body.

FIGS. 10A and 10B are a perspective view and schematic cross-sectional view, respectively, of a lead including a hydrogel fixation member extending around a perimeter of a lead body.

FIG. 11 is a perspective view of an implantable medical lead including hydrogel fixation members extending radially outward from a lead body at approximately 90° with respect to an outer surface of the lead body.

FIG. 12 is a flow diagram illustrating one example method for implanting a lead including hydrogel fixation member in accordance with an embodiment of the present invention.

### DETAILED DESCRIPTION

The present invention relates to an implantable medical elongated member including at least one expandable fixation member disposed in a recess defined by the elongated member, where the expandable fixation member is configured to expand upon implantation of the elongated member in a patient to substantially fix a position of the elongated member. The elongated member is configured to be coupled to a medical device to deliver a therapy from the medical device to target tissue in a patient. Various embodiments of the elongated member may be applicable to different therapeutic applications. For example, the elongated member may be a stimulation lead or lead extension that is used to deliver electrical stimulation to a target stimulation site and/or sense parameters (e.g., blood pressure, temperature or electrical activity) of a patient. In another embodiment, the elongated member may be a catheter that is placed to deliver a fluid, such as pharmaceutical agents, insulin, pain relieving agents, gene therapy agents or the like from a fluid delivery device (e.g., a fluid reservoir and/or pump) to a target tissue site in a patient. The invention is applicable to any configuration or type of implantable elongated member that is used to deliver therapy to a site in a patient. For purposes of illustration, however, the disclosure will refer to a neurostimulation lead.

In addition, the invention is applicable to any expandable fixation member, such as a hydrogel fixation member that expands upon absorption of a fluid or a shape memory fixation member that expands upon exposure to a particular temperature or another form of activation (e.g., electrical activation). The shape memory fixation member may be, for example, a fixation member formed from a shape memory metal alloy (e.g., Nitinol) or a shape memory polymer. For purposes of illustration, however, the disclosure will refer to a hydrogel fixation member.

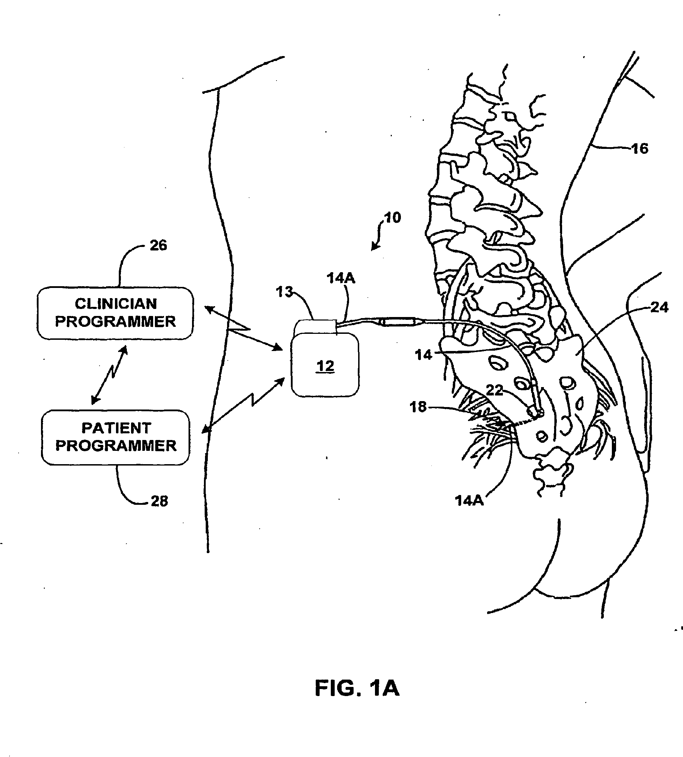

FIG. 1A a schematic perspective view of a therapy system 10, which includes electrical stimulator 12 coupled to implantable medical lead 14. Electrical stimulator 12 may be implantable or external. In the example of FIG. 1A, electrical stimulator 12 has been implanted in a body of patient 16 proximate to target stimulation site 18. For example, electrical stimulator 12 may be subcutaneously implanted in the body of a patient 16 (e.g., in a chest cavity, lower back, lower abdomen, or buttocks of patient 16). Electrical stimulator 12 provides a programmable stimulation signal (e.g., in the form of electrical pulses or a continuous signal) that is delivered to target stimulation site 18 by implantable medical lead 14, and more particularly, via one or more stimulation electrodes carried by lead 14. Electrical stimulator 12 may also be referred to as a signal generator, and in the embodiment shown in FIG. 1A, electrical stimulator 12 may also be referred to as a neurostimulator. In some embodiments, lead 14 may also carry one or more sense electrodes to permit neurostimulator 12 to sense electrical signals from target stimulation site 18. Furthermore, in some embodiments, neurostimulator 12 may be coupled to two or more leads, e.g., for bilateral or multi-lateral stimulation.

Proximal end 14A of lead 14 may be both electrically and mechanically coupled to connector 13 of neurostimulator 12 either directly or indirectly (e.g., via a lead extension). In particular, conductors disposed in the lead body may electrically connect stimulation electrodes (and sense electrodes, if present) located adjacent to distal end 14B of lead 14 to neurostimulator 12.

As described in further detail below, lead 14 further includes a lead body and at least one hydrogel fixation member (not shown in FIG. 1A) disposed in a recess defined by the lead body. The recess is typically sized to contain a large percentage or the entire hydrogel fixation member, which generally minimizes or eliminates the protrusion of the hydrogel fixation member past an outer surface of the lead body when the hydrogel fixation member is in a first state. Thus, the cross-sectional area of lead 14 is minimized when lead 14 is implanted in patient 16 and the hydrogel fixation member is in the first state. In an expanded state, the hydrogel fixation member protrudes out of the recess and extends from the lead body and engages with surrounding tissue to substantially fix a position of lead 14 proximate to target stimulation site 18.

Therapy system 10 may also include clinician programmer 26 and patient programmer 28. Clinician programmer 26 may be a handheld computing device that permits a clinician to program neurostimulation therapy for patient 16, e.g., using input keys and a display. For example, using clinician programmer 26, the clinician may specify neurostimulation parameters for use in delivery of neurostimulation therapy. Clinician programmer 26 supports telemetry (e.g., radio frequency (RF) telemetry) with neurostimulator 12 to download neurostimulation parameters and, optionally, upload operational or physiological data stored by neurostimulator 12. In this manner, the clinician may periodically interrogate neurostimulator 12 to evaluate efficacy and, if necessary, modify the stimulation parameters.

Like clinician programmer 26, patient programmer 28 may be a handheld computing device. Patient programmer 28 may also include a display and input keys to allow patient 16 to interact with patient programmer 28 and neurostimulator 12. In this manner, patient programmer 28 provides patient 16 with an interface for control of neurostimulation therapy by neurostimulator 12. For example, patient 16 may use patient programmer 28 to start, stop or adjust neurostimulation therapy. In particular, patient programmer 28 may permit patient 16 to adjust stimulation parameters such as duration, amplitude, pulse width and pulse rate, within an adjustment range specified by the clinician via clinician programmer 28, or select from a library of stored stimulation therapy programs.

Neurostimulator 12, clinician programmer 26, and patient programmer 28 may communicate via cables or a wireless communication, as shown in FIG. 1A. Clinician programmer 26 and patient programmer 28 may, for example, communicate via wireless communication with neurostimulator 12 using RF telemetry techniques known in the art. Clinician programmer 26 and patient programmer 28 also may communicate with each other using any of a variety of local wireless communication techniques, such as RF communication according to the 802.11 or Bluetooth specification sets, infrared communication, e.g., according to the IrDA standard, or other standard or proprietary telemetry protocols.

In the embodiment of therapy system 10 shown in FIG. 1A, target stimulation site 18 is proximate to the S3 sacral nerve, and lead 14 extends through the S3 sacral foramen 22 of sacrum 24 to access the S3 sacral nerve. Stimulation of the S3 sacral nerve may help treat pelvic floor disorders, urinary control disorders, fecal control disorders, interstitial cystitis, sexual dysfunction, and pelvic pain.

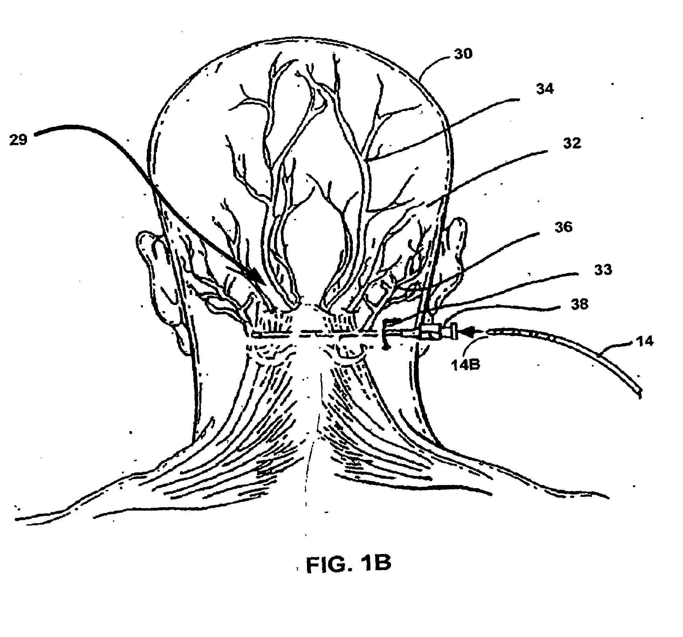

Therapy system 10, however, is useful in other neurostimulation applications. For example, as shown in FIG. 1B, lead 14 may be implanted and fixated with the hydrogel fixation members proximate to an occipital region 29 of patient 30 for stimulation of one or more occipital nerves. In particular, lead 14 may be implanted proximate to lesser occipital nerve 32, greater occipital nerve 34, and third occipital nerve 36. In FIG. 1B, lead 14 is aligned to be introduced into introducer needle 38 and implanted and anchored or fixated with fixation members proximate to occipital region 29 of patient 30 for stimulation of one or more occipital nerves 32, 34, and/or 36. A neurostimulator (e.g., neurostimulator 12 in FIG. 1A) may deliver stimulation therapy to any one or more of lesser occipital nerve 32, greater occipital nerve 34 or third occipital nerve 36 via electrodes disposed adjacent to distal end 14B of lead 14. In alternate embodiments, lead 14 may be positioned proximate to one or more other peripheral nerves proximate to occipital nerves 32, 34, and 36 of patient 30, such as nerves branching from occipital nerves 32, 34, and 36, as well as stimulation of any other suitable nerves throughout patient 30, such as, but not limited to, nerves within a brain, stomach or spinal cord of patient 30.

In the application of therapy system 10 shown in FIG. 1B, implantation of lead 14 may involve the subcutaneous placement of lead 14 transversely across one or more occipital nerves 32, 34, and/or 36 that are causing patient 30 to experience pain. In one example method of implanting lead 14 proximate to one or more occipital nerves 32, 34, and/or 36, a vertical skin incision 33 approximately two centimeters in length is made in the neck of patient 30 lateral to the midline of the spine at the level of the C1 vertebra. Typically, local anesthetic is used during the implantation procedure. The length of vertical skin incision 33 may vary depending on the particular patient. At this location, the patient's skin and muscle are separated by a band of connective tissue referred to as fascia. Introducer needle 38 is introduced into the subcutaneous tissue, superficial to the fascia and muscle layer but below the skin. Occipital nerves 32, 34, and 36 are located within the cervical musculature and overlying fascia, and as a result, introducer needle 38, and eventually lead 14, is inserted superior to occipital nerves 32, 34, and 36.

Once introducer needle 38 is fully inserted, lead 14 may be advanced through introducer needle 38 and positioned to allow stimulation of the lesser occipital nerve 32, greater occipital nerve 34, third occipital nerve 36, and/or other peripheral nerves proximate to an occipital nerve. Upon placement of lead 14, introducer needle 38 may be removed. As described below, when the one or more hydrogel fixation members of lead 14 are exposed to body fluids upon implantation in patient 30 (and upon withdrawal of introducer needle 38 in this case), hydrogel fixation members expand and extend from lead 14 to substantially fix a position of lead 14 proximate to one or more occipital nerves 32, 34, and/or 36.

Accurate lead placement may affect the success of occipital nerve stimulation. If lead 14 is located too deep, i.e. anterior, in the subcutaneous tissue, patient 30 may experience muscle contractions, grabbing sensations, or burning. Such problems may additionally occur if lead 14 migrates after implantation. Furthermore, due to the location of implanted lead 14 on the back of patient's 30 neck, lead 14 may be subjected to pulling and stretching that may increase the chances of lead migration. For these reasons, fixating lead 14 may be advantageous.

In alternate applications of lead 14, target stimulation site 18 may be a location proximate to any of the other sacral nerves in patient 16 or any other suitable nerve, organ, muscle or muscle group in patient 16, which may be selected based on, for example, a therapy program selected for a particular patient. For example, therapy system 10 may be used to deliver neurostimulation therapy to a pudendal nerve, a perineal nerve or other areas of the nervous system, in which cases, lead 14 would be implanted and substantially fixed proximate to the respective nerve. As further examples, lead 14 may be positioned for temporary or chronic spinal cord stimulation for the treatment of pain, for peripheral neuropathy or post-operative pain mitigation, ilioinguinal nerve stimulation, intercostal nerve stimulation, gastric stimulation for the treatment of gastric mobility disorders and obesity, muscle stimulation (e.g., functional electrical stimulation (FES) of muscles), for mitigation of other peripheral and localized pain (e.g., leg pain or back pain), or for deep brain stimulation to treat movement disorders and other neurological disorders. Accordingly, although patient 16 and target stimulation site 18 of FIG. 1A are referenced throughout the remainder of the disclosure for purposes of illustration, a neurostimulation lead 14 in accordance with the invention may be adapted for use in a variety of electrical stimulation applications, including occipital nerve stimulation, as shown in FIG. 1B with respect to patient 30.

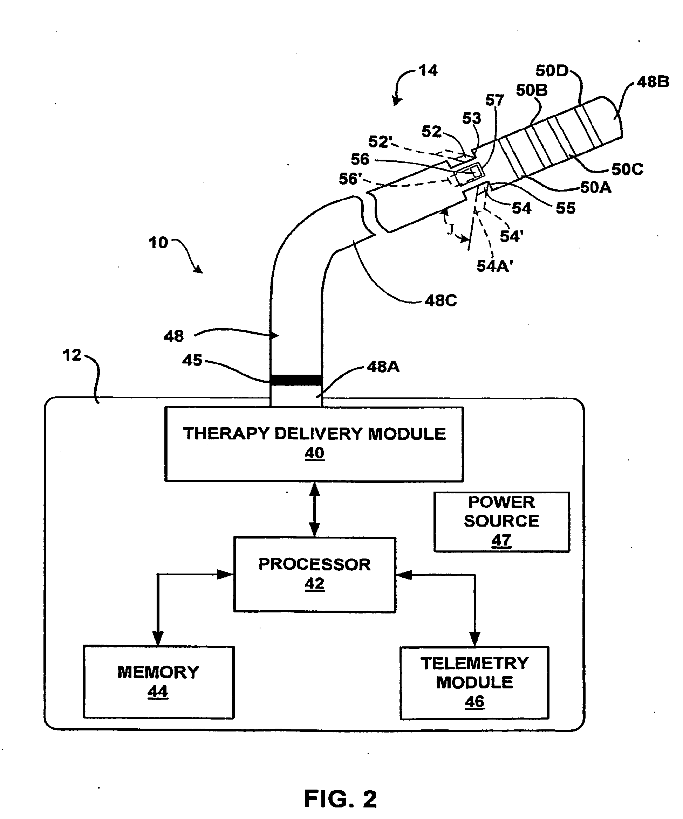

FIG 2 is a block diagram illustrating various components of neurostimulator 12 and an implantable medical lead 14. Neurostimulator 12 includes therapy delivery module 40, processor 42, memory 44, telemetry module 46, and power source 47. In some embodiments, neurostimulator 12 may also include a sensing circuit (not shown in FIG. 2). Implantable medical lead 14 includes lead body 48 extending between proximal end 48A and distal end 48B. Lead body 48 may be cylindrical or may be a paddle-shaped (i.e., a "paddle" lead). Electrodes 50A, 50B, 50C, and 50D (collectively "electrodes 50") are disposed on lead body 48 adjacent to distal end 48B of lead body 48. Proximal end 48A of lead body 48, includes contacts (not shown in FIG. 2) to electrically couple lead 14 (and in particular, electrodes 50) to a lead extension or neurostimulator 12.

In some embodiments, electrodes 50 may be ring electrodes. In other embodiments, electrodes 50 may be segmented or partial ring electrodes, each of which extends along an arc less than 360 degrees (e.g., 90-120 degrees) around the circumference of lead body 48.
In embodiments in which lead 14 is a paddle lead, electrodes 50 may extend along one side of lead body 48. The configuration, type, and number of electrodes 50 illustrated in FIG. 2 are merely exemplary.

Electrodes 50 extending around a portion of the circumference of lead body 48 or along one side of a paddle lead may be useful for providing an electrical stimulation field in a particular direction/targeting a particular therapy delivery site. For example, in the electrical stimulation application shown in FIG. 1B, electrodes 50 may be disposed along lead body 48 such that the electrodes face toward occipital nerves 32, 34, and/or 36, or otherwise away from the scalp of patient 30. This may be an efficient use of stimulation because electrical stimulation of the scalp may not provide any therapy to patient 30. In addition, the use of segmented or partial ring electrodes 50 may also reduce the overall power delivered to electrodes 50 by neurostimulator 12 because of the efficient delivery of stimulation to occipital nerves 32, 34, and/or 36 (or other target stimulation site) by eliminating or minimizing the delivery of stimulation to unwanted or unnecessary regions within patient 30.

In embodiments in which electrodes 50 extend around a portion of the circumference of lead body 48 or along one side of a paddle lead, lead 14 may include one or more orientation markers 45 proximate to proximal end 14A that indicate the relative location of electrodes 50. Orientation marker 45 may be a printed marking on lead body 48, an indentation in lead body 48, a radiographic marker, or another type of marker that is visible or otherwise detectable (e.g., detectable by a radiographic device) by a clinician. Orientation marker 45 may help a clinician properly orient lead 14 such that electrodes 50 face the desired direction (e.g., toward occipital nerves 32, 34, and/or 36) within patient 16. For example, orientation marker 45 may also extend around the same portion of the circumference of lead body 48 or along the side of the paddle lead as electrodes 50. In this way, orientation marker 45 face the same direction as electrodes, thus indicating the orientation of electrodes 50 to the clinician. When the clinician implants lead 14 in patient 16, orientation marker 45 may remain visible to the clinician.

Neurostimulator 12 delivers stimulation therapy via electrodes 50 of lead 14. In particular, electrodes 50 are electrically coupled to a therapy delivery module 40 of neurostimulator 12 via conductors within lead body 48. In one embodiment, an implantable signal generator or other stimulation circuitry within therapy delivery module 40 delivers electrical signals (e.g., pulses or substantially continuous signals, such as sinusoidal signals) to targets stimulation site 18 (FIG. 1 A) via at least some of electrodes 50 under the control of a processor 42. The implantable signal generator may be coupled to power source 47. Power source 47 may take the form of a small, rechargeable or non-rechargeable battery, or an inductive power interface that transcutaneously receives inductively coupled energy. In the case of a rechargeable battery, power source 47 similarly may include an inductive power interface for transcutaneous transfer of recharge power.

The stimulation energy generated by therapy delivery module 40 may be formulated as neurostimulation energy, e.g., for treatment of any of a variety of neurological disorders, or disorders influenced by patient neurological response. The pulses may be delivered from therapy delivery module 40 to electrodes 50 via a switch matrix and conductors carried by lead 14 and electrically coupled to respective electrodes 50.

Processor 42 may include a microprocessor, a controller, a DSP, an ASIC, an FPGA, discrete logic circuitry, or the like. Processor 42 controls the implantable signal generator within therapy delivery module 40 to deliver neurostimulation therapy according to selected stimulation parameters. Specifically, processor 42 controls therapy delivery module 40 to deliver electrical pulses with selected amplitudes, pulse widths, and rates specified by the programs. In addition, processor 42 may also control therapy delivery module 40 to deliver the neurostimulation pulses via selected subsets of electrodes 50 with selected polarities. For example, electrodes 50 may be combined in various bipolar or multi-polar combinations to deliver stimulation energy to selected sites, such as nerve sites adjacent the spinal column, pelvic floor nerve sites, or cranial nerve sites.

Processor 42 may also control therapy delivery module 40 to deliver each pulse according to a different program, thereby interleaving programs to simultaneously treat different symptoms or provide a combined therapeutic effect. For example, in addition to treatment of one symptom such as sexual dysfunction, neurostimulator 12 may be configured to deliver neurostimulation therapy to treat other symptoms such as pain or incontinence.

Memory 44 of neurostimulator 12 may include any volatile or non-volatile media, such as a RAM, ROM, NVRAM, EEPROM, flash memory, and the like. In some embodiments, memory 44 of neurostimulator 12 may store multiple sets of stimulation parameters that are available to be selected by patient 16 via patient programmer 28 (FIG. 1A) or a clinician via clinician programmer 26 (FIG. 1A) for delivery of neurostimulation therapy. For example, memory 44 may store stimulation parameters transmitted by clinician programmer 26 (FIG. 1A). Memory 44 also stores program instructions that, when executed by processor 42, cause neurostimulator 12 to deliver neurostimulation therapy. Accordingly, computer-readable media storing instructions may be provided to cause processor 42 to provide functionality as described herein.

In particular, processor 42 controls telemetry module 46 to exchange information with an external programmer, such as clinician programmer 26 and/or patient programmer 28 (FIG. 1A), by wireless telemetry. In addition, in some embodiments, telemetry module 46 supports wireless communication with one or more wireless sensors that sense physiological signals and transmit the signals to neurostimulator 12.

As previously discussed, migration of lead 14 following implantation may be undesirable, and may have detrimental effects on the quality of therapy delivered to a patient 16. For example, with respect to the sacral nerve stimulation application shown in FIG. 1A, migration of lead 14 may cause displacement of electrodes carried adjacent to distal end 14B of lead 14 with respect to target stimulation site 18. In such a situation, the electrodes may not be properly positioned to deliver therapy to target stimulation site 18, resulting in reduced electrical coupling, and possibly undermining therapeutic efficacy of the neurostimulation therapy from system 10. Substantially fixing lead 14 to surrounding tissue may help prevent lead 14 from migrating from target stimulation site 18 following implantation, which may ultimately help avoid harmful effects that may result if implantable medical lead 14 migrates from target stimulation site 18.

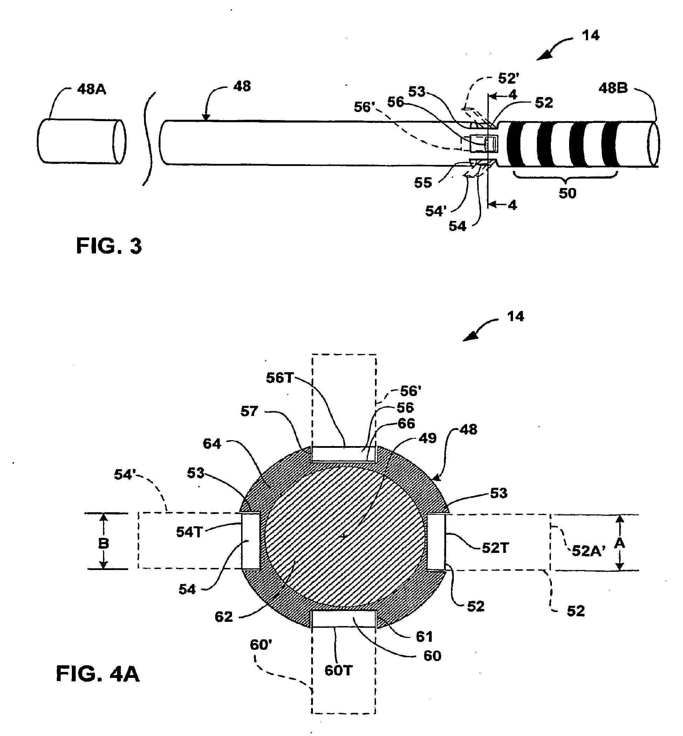

To that end, lead 14 includes hydrogel fixation members 52, 54, and 56 that are attached to lead body 48 to fix lead 14 to tissue surrounding lead 14, such as tissue within sacrum 24 in the example of FIG. 1A or tissue at occipital region 29 in the example of FIG. 1B. In the embodiment shown in FIG. 2, hydrogel fixation members 52, 54, and 56 share an axial location along lead body 48. Hydrogel fixation members 52, 54, and 56 expand upon implantation in patient 16 (FIG. 1A) to engage with surrounding tissue. Hydrogel fixation members 52, 54, and 56 are positioned on lead body 48 between proximal end 48A of lead body 48 and electrodes 50. In particular, hydrogel fixation member 52 is disposed in recess 53 in lead body 48, hydrogel fixation member 54 is disposed in recess 55, and hydrogel fixation member 56 is disposed in recess 57. Lead 14 also includes a fourth hydrogel fixation member 60 disposed in a recess in lead body 48 on an opposite side lead body 48 from hydrogel fixation member 56, which is not shown in FIG. 2, but is shown in FIG. 4A.

While hydrogel fixation members 52, 54, 56, and 60 do not necessarily restrict all motion of lead 14 when hydrogel fixation members 52, 54, 56, and 60 are in an expanded state, hydrogel fixation members 52, 54, 56, and 60 generally reduce the motion of lead 14 so that lead 14 remains proximate to target nerve site 18. In comparison to some existing methods of fixing implanted medical leads, such as suturing lead 14 to surrounding tissue, hydrogel fixation members 52, 54, 56, and 60 of the invention may permit implantation of lead 14 in patient 16 via a minimally invasive surgery, which may allow for reduced pain and discomfort for patient 16 relative to surgery, as well as a quicker recovery time. As previously mentioned, hydrogel fixation members 52, 54, 56, and 60 are disposed in recesses 53, 55, 57, and 61 respectively, in lead body 48, which enables lead 14 to maintain a relatively small profile during implantation in patient 16, thereby further minimizing the invasiveness of the implantation procedure. In addition, hydrogel fixation members 52, 54, 56, and 60 are generally self-deploying upon exposure to body fluids, which may help streamline an implantation procedure.

Hydrogel fixation members 52, 54, 56, and 60 are configured to expand from a first dimension in a in a first state (e.g., a dehydrated or substantially dehydrated state) to a second dimension in a second state (e.g., a state that is more hydrated than the first state, and may thus be referred to as a "hydrated state") to engage with surrounding tissue. The first state of hydrogel fixation members 52, 54, and 56 is shown in solid lines in FIG. 2, and the second state is shown in phantom lines and indicated by hydrogel fixation members 52', 54', and 56'. The first state of hydrogel fixation members 52, 54, 56, and 60 accommodates relatively minimally invasive implantation of lead 14 in patient 16 (FIG 1A) because of the relatively small profile of hydrogel fixation members 52, 54, 56, and 60 in the unexpanded state and also because hydrogel fixation members 52, 54, 56, and 60 are disposed in recesses 53, 55, 57, and 61.

Hydrogel fixation members 52, 54, 56, and 60 expand to the second state upon absorption of water from the surrounding body tissue. Otherwise stated, each hydrogel fixation member 52, 54, 56, and 60 in accordance with the invention assumes an expanded, hydrated state after absorbing a sufficient amount of fluid from patient 16 after implantation in patient 16, thereby substantially fixing a position of lead 14. In the expanded state, hydrogel fixation members 52, 54, 56, and 60 protrude out of the respective recess 53, 55, 57, and 61 in lead body 48 and extend from lead body 48.

Hydrogel fixation members 52, 54, 56, and 60 continue to expand until hydrogel fixation members 52, 54, 56, and 60 are saturated with fluid, or until surrounding tissue exerts an equal amount of pressure on hydrogel fixation members 52, 54, and 56, thereby preventing hydrogel fixation members 52, 54, 56, and 60 from further expanding. In one embodiment, hydrogel fixation members 52, 54, 56, and 60 expand to at least two to five times their unexpanded (i.e., the first dimension) size. In this way, hydrogel fixation members 52, 54, 56, and 60 generally self-deploy upon implantation in patient 16. If desired, a fluid may be introduced into patient 16 at or near target stimulation site 18 in order to accelerate the expansion of hydrogel fixation members 52, 54, and 56. In the hydrated state, lead body 48 has a greater profile because hydrogel fixation members 52, 54, 56, and 60 extend from lead body 48 to engage with surrounding tissue and substantially fix a position of lead 14 and, in particular, substantially fix a position of electrodes 50 proximate to a target stimulation site 18 (FIG. 1A).

The "dehydrated" and "hydrated" states are relative to each other. For example, as used herein, "dehydrated" does not necessarily mean that the polymeric matrix composing hydrogel fixation members 52, 54, 56, and 60 is 100% devoid of fluid, but that the polymeric matrix is more devoid of fluid than in the "hydrated" state. Or from the perspective of the hydrated state, "hydrated" does not necessarily mean that the polymeric matrix is saturated with fluid, but rather that the hydrated member 52, 54, 56 includes more fluid than in the "dehydrated state."

The expansion of hydrogel fixation members 52, 54, 56, and 60 is generally in a radially outward direction, but in some embodiments, the expansion includes both an axial and radial component because hydrogel fixation members 52, 54, 56, and 60 may extend from lead body 48 at angle J when hydrogel fixation members 52, 54, 56, and 60 are in an expanded state, where angle J is measured between longitudinal surface 54A' of the expanded hydrogel fixation member 54 and outer surface 48C of lead body 48. In the embodiment shown in FIG 2, angle J is less than about 90°. In other embodiments, however, angle J may be approximately equal to or greater than about 90°. Each of hydrogel fixation members 52, 54, 56, and 60 may or may not extend from lead body 48 at the same angle with respect to outer surface 48C of lead body 48. Hydrogel fixation members 52, 54, 56, and 60 may be formed to extend from lead body 48 at angle J using any suitable method of forming hydrogel structures.

For example, in one method, hydrogel fixation members 52, 54, 56, and 60 may be initially formed to have a first shape, such as a rectangle having a plane that extends orthogonally from outer surface 48C of lead body 48 (e.g., surface 54A' is generally perpendicular to outer surface 48C), in an expanded and hydrated state. While hydrogel fixation members 52, 54, 56, and 60 are still in the expanded state, the first shape may be cut, molded or otherwise shaped to form hydrogel tines, flanges or other protrusions that extends from lead body 48 at angle J with respect to outer surface 48C of lead body 48. Hydrogel fixation members 52, 54, 56, and 60 may be subsequently desiccated to the unexpanded, dehydrated state prior to implantation in patient 16 (FIG. 1A). In some embodiments, hydrogel fixation members 52, 54, 56, and 60 may be trimmed after desiccation if hydrogel fixation members 52, 54, 56, and 60 protrude past outer surface 48C of lead body 48.

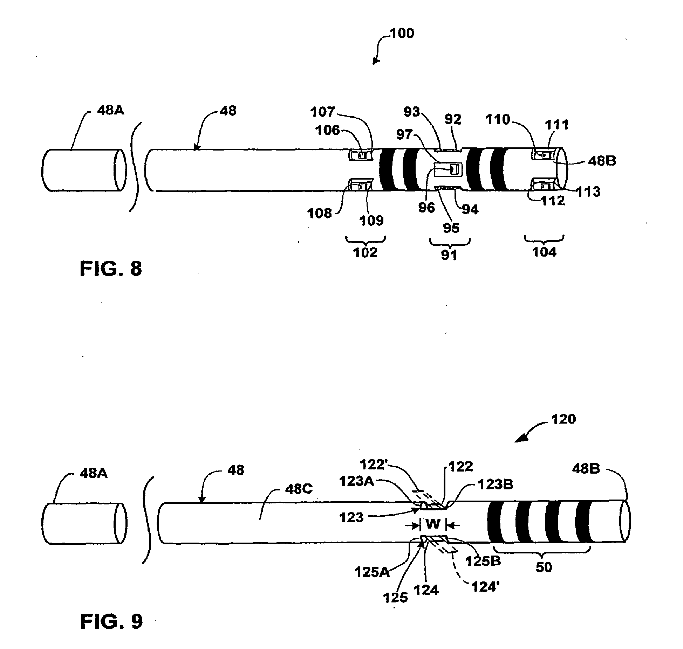

In some applications of lead 14, it may be desirable for hydrogel fixation members 52, 54, 56, and 60 to extend from lead body 48 at angle J toward proximal end 48A of lead body 48 in order to help secure lead 14 in place. Angled hydrogel fixation members 52, 54, 56, and 60 may help lead 14 resist both axial and radial movement. For example, in therapy system 10, distal end 48B of lead body 48, which is adjacent to electrodes 50, may be inclined to pull toward neurostimulator 12 because proximal end 48A of lead body 48 is mechanically coupled to neurostimulator 12. Hydrogel fixation members 52, 54, 56, and 60 that are angled toward proximal end 48A of lead body 48 (as shown in FIG. 2) may help distal end 48B of lead body 48 resist the pulling force from proximal end 48A. Of course, it may be desirable in some applications for hydrogel fixation members 52, 54, 56, and 60 to extend toward distal end 48B of lead body 48. Accordingly, the invention contemplates configurations of hydrogel fixation members 52, 54, 56, and 60 that are angled both toward and away from proximal end 48A of lead body 48 (e.g., as shown in FIG 9).

During implantation, hydrogel fixation members 52, 54, 56, and 60 may be restrained from expansion (i.e., remain in the first state) by substantially preventing hydrogel fixation members 52, 54, 56, and 60 from being exposed to fluids. For example, during implantation, hydrogel fixation members 52, 54, 56, and 60 may be separated from fluids until lead 14 is located proximate to target stimulation site 18 by separating hydrogel fixation members 52, 54, 56, and 60 from surrounding tissue via an introducer needle, a sheath, or a substantially water impermeable, biodegradable coating. Upon deployment from the introducer, hydrogel fixation members 52, 54, 56, and 60 contact surrounding tissue, which carry fluid (e.g., water in the blood), and hydrogel fixation members 52, 54, 56, and 60 expand. The ability to separate hydrogel fixation members 52, 54, 56, and 60 from fluid until implantation in patient 16 permits lead 14 to maintain a relatively small lead profile (e.g., an overall lead diameter) during lead insertion via a needle. Despite the relatively small overall lead diameter, lead 14 is able to carry a fixation mechanism that extend from lead 14 because hydrogel fixation members 52, 54, 56, and 60 assume an expanded state once lead 14 is implanted in patient 16, distal end 14B of lead 14 is deployed from the introducer needle, and hydrogel fixation members 52, 54, 56, and 60 absorb a sufficient amount of fluid from tissue near target stimulation site 18.

Hydrogel fixation members 52, 54, and 56 are shown in FIG. 2 as having substantially quadrilateral shapes. However, in other embodiments, hydrogel fixation members 52, 54, and 56 (as well as hydrogel fixation member 60) may assume any suitable shaped protrusion suitable for engaging with surrounding tissue to substantially fix a position of lead 14 such that electrodes 50 remain proximate to target stimulation site 18 (FIG. 1A). For example, in one embodiment, hydrogel fixation members 52, 54, 56, and 60 have rounded edges.

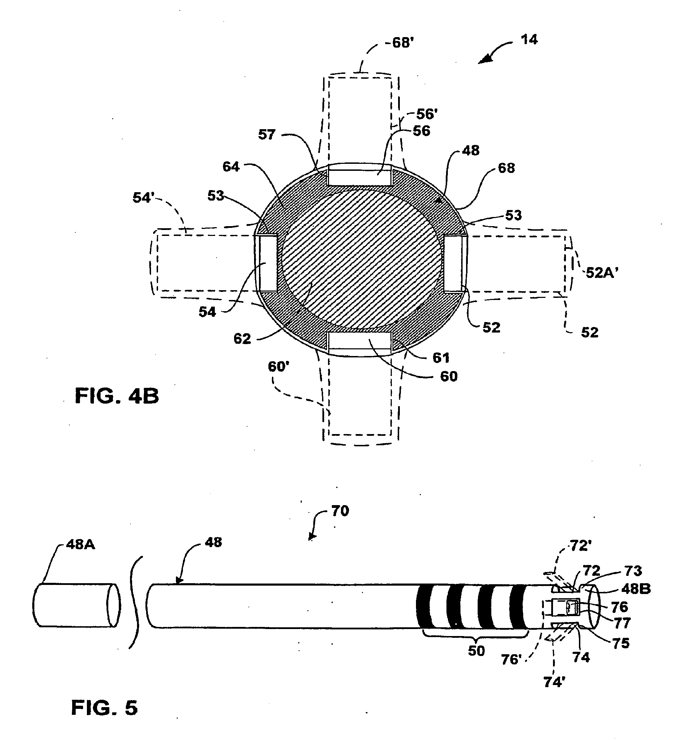

In alternate embodiments, hydrogel fixation members 52, 54, 56, and 60 may have other arrangements along lead body 48 that enable fixation members 52, 54, 56, and 60 to substantially fix electrodes 50 proximate to target stimulation site 18. For example, hydrogel fixation members 52, 54, 56, and 60 may have different axial locations along lead body 48 (i.e., at least one of hydrogel fixation members 52, 54, 56, and 60 may be offset from line 58). As another example of an alternate arrangement, one or more fixation members 52, 54, 56, and 60 may be located between electrodes 50 and distal end 48B of lead body 48. Alternatively, at least one hydrogel fixation member 52, 54 or 56 may be located between two or more electrodes 50. Although FIG. 2 illustrates lead 14 including three hydrogel fixation members 52, 54, and 56, in alternate embodiments, lead 14 may include any suitable number of hydrogel fixation members. These and other embodiments of leads including alternate numbers and/or arrangements of hydrogel fixation members are shown in FIGS. 5-11 and described in reference thereto. In FIGS. 1A-11, the components of the leads, as well I as any other components that may be illustrated, are not necessarily drawn to scale. For example, each of the hydrogel fixation members 52, 54, 56, and 60 shown in FIGS. 2-4 are not necessarily drawn in correct proportion to the length or diameter of lead body 48.

Hydrogel fixation members 52, 54, 56, and 60 may each be composed of any suitable biocompatible hydrogel material, which is typically a network of polymer chains that expand upon absorbing fluid, and in this case, such fluid may be water or blood from tissue. Examples of suitable hydrogel materials include, but are not limited to, but not limited to a pure hydrogel, a blend of silicone rubber and hydrogel, polyacrylonitrile copolymers or a polymeric matrix including an osmotic agent. The particular type of hydrogel (e.g., amount of cross-linking between the polymer chains forming the hydrogel) or quantity of pure hydrogel in the mixture is selected to provide a desired amount of expansion of hydrogel fixation members 52, 54, 56, and 60 and the desired degree of flexibility or rigidity of hydrogel fixation members 52, 54, 56, and 60 in an expanded state.

In some embodiments, the material forming hydrogel fixation members 52, 54, 56, and 60 is also selected such that hydrogel fixation members 52, 54, 56, and 60 assume an expanded state within a desired time (e.g., one to ten minutes after exposure to body fluids). The control of the timing of deployment of hydrogel fixation members 52, 54, 56, and 60 allow a clinician sufficient time to guide the lead through patient 16 and into a desired position proximate to the target stimulation site 18 within sacrum 24 or otherwise. The rate of deployment may be further controlled by applying a coating of a soluble material, such as mannitol, which dissolves before the hydrogel begins to hydrate.

Hydrogel fixation members 52, 54, 56, and 60 may also be attached to lead body 48 such that hydrogel fixation members 52, 54, 56, and 60 are configured to break away from lead body 48. For example, hydrogel fixation members 52, 54, 56, and 60 may be attached to lead body 48 with an adhesive that loses its adhesive properties over time, which results in hydrogel fixation members 52, 54, 56, and 60 breaking away from lead body 48 as a consequence of a passing of a certain amount of time. For example, hydrogel fixation members 52, 54, 56, and 60 may be attached to lead body 48 with a resorbable adhesive that eventually releases hydrogel fixation members 52, 54, 56, and 60 after a sufficient period of time for tissue to encapsulate lead body 48. After tissue ingrowth, hydrogel fixation members 52, 54, 56, and 60 may not be necessary to substantially fix lead 14 in place. In this embodiment, hydrogel fixation members 52, 54, 56, and 60 may be formed of a biodegradable hydrogel, such as polyvinylalcohol, polyethyleneoxide, and hydrogel multiblock polymers, such that body of patient 16 is able to absorb and decompose hydrogel fixation members 52, 54, 56, and 60 after breaking off of lead body 48.

Alternatively, hydrogel fixation members 52, 54, 56, and 60 may be configured to break away from lead body 48 as a consequence of a purposeful removal of hydrogel fixation members 52, 54, 56, and 60 from lead body 48. For example, hydrogel fixation members 52, 54, 56, and 60 may be attached to lead body 48 with an adhesive and purposefully removed therefrom by introducing a solvent into patient 16 to dissolve the adhesive or otherwise diminish the adhesive properties of the adhesive. Embodiments of lead 14 including hydrogel fixation members 52, 54, 56, and 60 that are capable of breaking away from lead body 48 may be useful for explanting lead 14 from patient 16. This feature may also enable hydrogel fixation members 52, 54, 56, and 60 to temporarily fix electrodes 50 of lead 14 proximate to stimulation site 18 until tissue encapsulates lead body 48 to fix lead 14 in place.

In another embodiment, hydrogel fixation members 52, 54, 56, and 60 may be otherwise configured to permit explant of lead 14 without undue force or without damaging surrounding tissue. For example, hydrogel fixation members 52, 54, 56, and 60 may be formed of material having a durometer that allows adequate fixation of lead 14 at target stimulation site 18, but also allows removal of lead 14 upon the application of a strong removal force. When subjected to a force higher than the application force (i.e., the particular forces attributable to the movement of patient 16, which fixation members 52, 54, 56, and 60 withstand at target stimulation site 18 to substantially fix a position of lead 14), fixation members 52, 54, 56, and 60 yield and collapse relatively close to outer surface 48C of lead body 48 to permit explantation of lead 14 without undue force or breakage of lead 14.

FIG 3 is a perspective view of implantable medical lead 14 of FIGS. 1A, 1B, and 2, which includes hydrogel fixation members 52, 54, 56, and 60 to fix lead 14 to surrounding tissue to help prevent lead migration following implantation proximate to target stimulation site 18 (FIG. 1A). As described above, lead 14 includes lead body 48 extending from proximal end 48A to distal end 48B, array of electrodes 50, and hydrogel fixation members 52, 54, 56, and 60 disposed in recesses 53, 55, 57, and 61, respectively. As FIG. 3 illustrates, when hydrogel fixation members 52, 54, and 56 are in an unexpanded state, lead 14 has a relatively small profile because hydrogel fixation members 52, 54, and 56 do not extend past outer surface 48C of lead body 48, thereby minimizing the profile of lead 14 when hydrogel fixation members 52, 54, and 56 are in the unexpanded state. This may be useful, for example, during implantation of lead 14 in patient 16 (FIG 1A) because a smaller diameter introducer needle may be used, which may help minimize the invasiveness of the implantation procedure. An introducer needle that has a lumen diameter sized to accommodate lead body 48 may be used rather than an introducer having a greater lumen diameter that is sized to accommodate lead body 48 with hydrogel fixation members that sit on outer surface 48C.

When hydrogel fixation members 52, 54, and 56 are in an expanded state (as shown by phantom lines and indicated as hydrogel fixation members 52', 54', and 56'), lead 14 has a greater profile. The greater profile of lead 14 upon expansion of fixation members 52, 54, and 56 enables fixation members to engage with surrounding tissue upon implantation in patient 16.

FIG. 4A is a schematic cross-sectional view of lead 14 taken along line 4-4 in FIG. 3 and illustrates hydrogel fixation members 52, 54, 56, and 60 disposed in recesses 53, 55, 57, and 61, respectively, in lead body 48. In the embodiment shown in FIG. 4A, top surface 52T, 54T, 56T, and 60T of each hydrogel fixation member 52, 54, 56, and 60, respectively, is generally flush with outer surface 48C of lead body 48. However, in other embodiments, top surfaces 52T, 54T, 56T, and 60T may protrude past outer surface 48C of lead body 48 or may be below outer surface 48C of lead body 48.

Lead body 48 carries a plurality of conductors 62 (shown in FIG. 4A as a single conductive center of lead body 48 for clarity of illustration) for electrically coupling electrodes 50 (FIG. 3) to therapy delivery module 40 of neurostimulator 12 (FIG. 2). Typically, a separate conductor electrically couples each electrode 50A-D to therapy delivery module 40. Separate conductors permit independent selection of individual electrodes 50A-D. Furthermore, each of the conductors electrically coupled to separate electrodes 50A-D are electrically insulated from each other. Insulating layer 64 surrounds conductors 60 in order to electrically insulate conductors 60 from tissue when lead 14 is implanted in patient 16 and from a clinician when the clinician is implanting lead 14 in a patient.

In the embodiment of FIGS. 3 and 4, recesses 53, 55, 57, and 61 are formed in insulating layer 64 using any suitable method. For example, insulating layer 64 may be molded, embossed, etched, cut or milled to form recesses 53, 55, 57, and 61. Recesses 53, 55, 57, and 61 are shown in FIG. 4A as having rectangular shapes. However, in alternate embodiments, recesses 53, 55, 57, and 61 may have any suitable shape, which may depend upon the shape of hydrogel fixation members 52, 54, 56, and 60, because recesses 53, 55, 57, and 61 are typically sized and shaped to accommodate hydrogel fixation members 52, 54, 56, and 60. Recesses 53, 55, 57, and 61 and the other components of lead 14 are not necessarily drawn to scale in FIGS. 2, 3, and 4. In another embodiment, two or more hydrogel fixation members 52, 54, 56, and 60 may share a recess rather than in individual recesses.

In the embodiment shown in FIG. 4A, lead 14 includes four hydrogel fixation members 52, 54, 56, and 60 equally spaced around an outer perimeter of lead body 48. Lead 14 may include any suitable number of hydrogel fixation members for substantially fixing electrodes 50 (FIG. 3) proximate to target stimulation site 18 (FIG. 1A). In addition, hydrogel fixation members 52, 54, 56, and 60 may be arranged in any suitable fashion about lead body 48. For example, hydrogel fixation members 52, 54, 56, and 60 need not be disposed around the entire outer perimeter of lead body 48 (e.g., may only include hydrogel fixation members 52, 54, and 56).

Although hydrogel fixation members 52, 54, 56, and 60 are shown in FIGS. 2-4 to be evenly spaced about an outer perimeter of lead body 48, in alternate embodiments, hydrogel fixation members 52, 54, 56, and 60 may be unevenly spaced about the outer perimeter of lead body 48. That is, hydrogel fixation members 52, 54, 56, and 60 may be asymmetrically distributed about the outer perimeter of lead body 48. For example, hydrogel fixation member 52 may be closer to hydrogel fixation member 56 than hydrogel fixation member 54. Furthermore, in alternate embodiments, hydrogel fixation members 52, 54, 56, and 60 are not the same size, but may be different sizes. For example, in one embodiment, dimension A of an expanded hydrogel fixation member 52' may greater than dimension B of an expanded hydrogel fixation member 54'. In another embodiment, dimension B of expanded hydrogel fixation member 54' may be greater than dimension A of expanded hydrogel fixation member 52'. In some embodiments, it may be useful to select the size of each of hydrogel fixation members 52, 54, 56, and 60 based on the particular application of therapy system (FIG. 2). In particular, it may be desirable to select the size of or otherwise configure hydrogel fixation members 52, 54, 56, and 60 to fix lead 14 to a particular region of the patient proximate to the target stimulation site (e.g., a peripheral nerve stimulation site), which may involve select the size of hydrogel fixation members 52, 54, 56, and 60 to accommodate the specific anatomical configuration of a region of the patient proximate to the peripheral nerve.

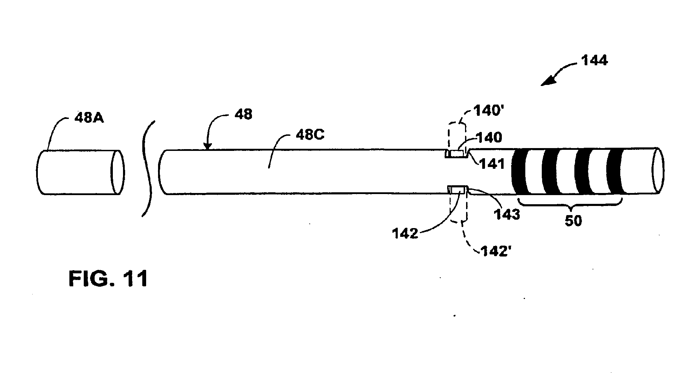

Hydrogel fixation members 52, 54, 56, and 60 may define any suitable shaped protrusion, such as a tine or flange-like structure, which is capable of engaging with surrounding tissue when in an expanded state. In accordance with the invention, hydrogel fixation members 52, 54, 56, and 60 may have any suitable cross-sectional shape, such as, but not limited to, a wedge shape, a rectangular shape, a curvilinear shape, and so forth. The shape should be selected such that hydrogel fixation members 52, 54, 56, and 60 are capable of extending from lead body 48 and engaging with surrounding tissue. For example, in the embodiment shown in FIG 4A, hydrogel fixation members 52, 54, 56, and 60 have a rectangular profile in an unexpanded state. In an expanded state (as indicated in phantom lines in FIG. 4A and identified as hydrogel fixation members 52', 54', 56', and 60'), hydrogel fixation members 52, 54, 56, and 60 each have a rectangular profile. In another embodiment, hydrogel fixation members 52, 54, 56, and 60 may have a curvilinear profile in an unexpanded and/or expanded state, as shown in FIG. 11. The curved edge may provide a relatively blunt edge for contacting surrounding tissue when lead 14 is implanted in patient 16 (FIG. 1A).

A cross-sectional shape of hydrogel fixation members 52, 54, 56, and 60, where a cross-section is taken substantially perpendicular to a longitudinal axis 49 of lead body 48, as shown in FIG. 4A, may be defined using any suitable method. Longitudinal axis 49 of lead body 48 is substantially perpendicular to the plane of the image of FIG. 4A. In one method, hydrogel fixation members 52, 54, 56, and 60 may be shaped (e.g., in a rectangular shape with a curved edge) when hydrogel fixation members 52, 54, 56, and 60 are in an expanded state, as described above with reference to an example method for forming hydrogel fixation members 52, 54, 56, and 60 to extend from lead body 48 at angle J. Hydrogel fixation members 52, 54, 56, and 60 may then subsequently be desiccated to the unexpanded, dehydrated state. Hydrogel fixation members 52, 54, 56, and 60 may be molded or cut into the desirable shape.

Hydrogel fixation members 52, 54, 56, and 60 may be attached to lead body 48, and more specifically, in recesses 53, 55, 57, and 61, respectively, defined by lead body 48, using any suitable attachment means. For example, hydrogel fixation members 52, 54, 56, and 60 may be attached to lead body 48 with a suitable biocompatible adhesive. For example, in the embodiment of lead 14 shown in FIG 4A, adhesive layer 66 attaches hydrogel fixation member 56 to insulating layer 64 of lead body 48. Adhesive layer 66 and lead 14 are not necessarily drawn to scale.

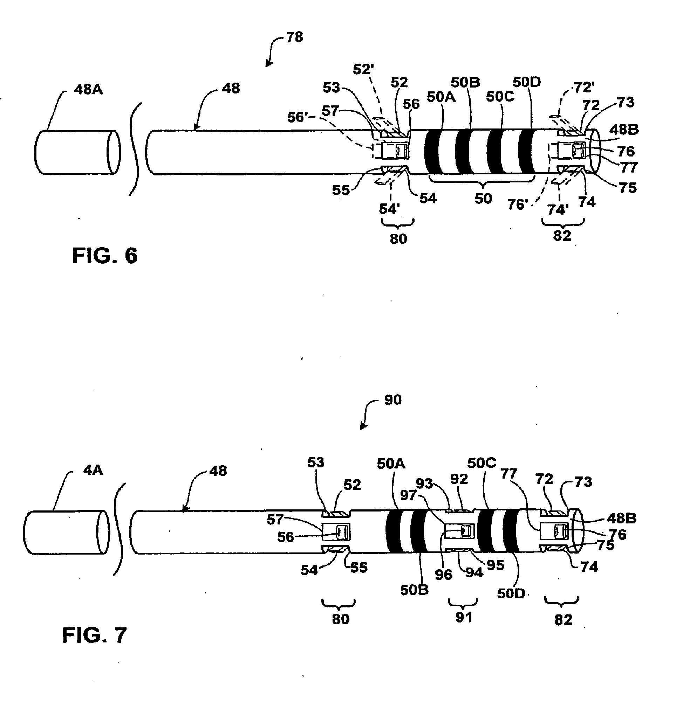

Another fixation means is shown in FIG. 4B, which shows the schematic cross-sectional view of lead 48 shown in FIG. 4, as well as expandable sleeve 68 disposed around lead body 48 to cover recesses 53, 55, 57, and 61 and contain hydrogel fixation members 52, 54, 56, and 60, respectively, within recesses 53, 55, 57, and 61. Adhesive 66 is not shown in FIG. 4B. Expandable sleeve 68 may be a continuous piece of a water-permeable and biocompatible elastic or other expandable material that is configured to fit around lead body 48. For example, expandable sleeve 68 may be comprised of silicone. Expandable sleeve 68 may be fitted around lead body 48 by introducing distal end 48B (FIG. 6) of lead body 48 into expandable sleeve 68. In some embodiments, expandable sleeve 68 may be the only means of attaching hydrogel fixation members 52, 54, 56, and 60 to lead body 48. Alternatively, an additional attachment means, such as an adhesive, may be used.

Upon implantation of lead 48 in patient 16 (FIG. 1), fluid from surrounding tissue permeates through expansion sleeve 68 to hydrogel fixation members 52, 54, 56, and 60, which expand upon absorption of the fluid. As FIG. 4B illustrates, portions of expandable sleeve 68 adjacent to hydrogel fixation members 52, 54, 56, and 60 expand with hydrogel fixation members 52, 54, 56, and 60 as hydrogel fixation members 52, 54, 56, and 60 expand from a first state to a second, hydrated state. The expansion of expansion sleeve 68 is indicated in phantom lines in FIG. 4B and indicated as expansion sleeve 68'.

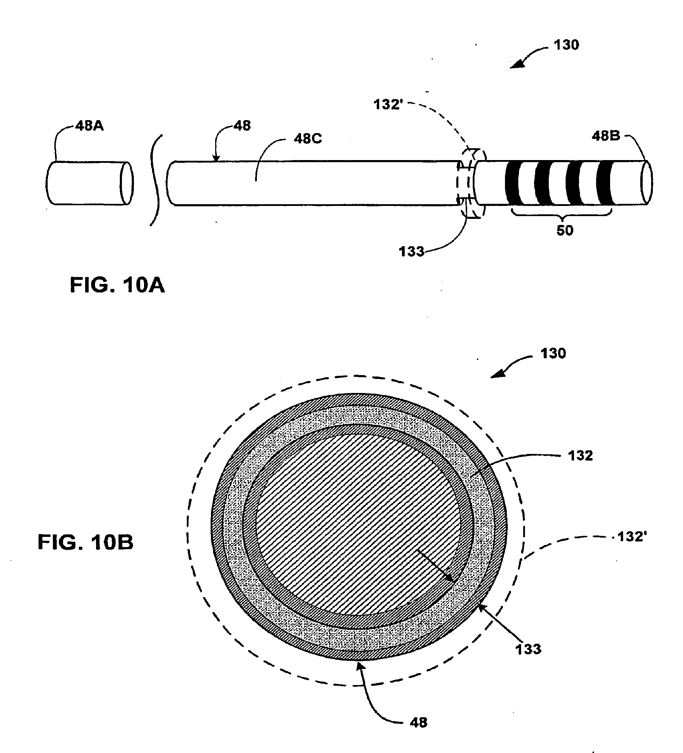

FIGS. 5-9 illustrate alternate arrangements of hydrogel fixation members on a lead body. FIGS. 10 and 11 illustrate alternate shapes for hydrogel fixation members. For clarity of description, like numbered reference numbers designate substantially similar elements throughout FIGS. 2-11.

FIG. 5 is a perspective view of lead 70, which includes hydrogel fixation members 72, 74, and 76 disposed in recesses 73, 75, and 77, respectively, proximate to distal end 48B of lead body 48. A fourth hydrogel fixation member (not shown) may be disposed along lead body 48 opposite hydrogel fixation member 76 and extending into the plane of the image of FIG. 5. Hydrogel fixation members 72, 74, and 76, as well as their respective recesses 73, 75, and 77 are substantially similar in structure to hydrogel fixation members 52, 54, 56, and 60 of FIGS. 2-4B, but differ from hydrogel fixation members 52, 54, 56, and 60 in that hydrogel fixation members 72, 74, and 76 are placed between electrodes 50 and distal end 48B of lead body 48 rather than between electrodes 50 and proximal end 48A. While both leads 14 and 70 include hydrogel fixation members for fixing leads 14 and 70, respectively, to target stimulation site 18, lead 70 may be useful for locally fixing distal end 48B of lead body 48. In some applications of therapy system 10 (FIGS. 1A and 2), such as when therapy system 10 is used to stimulate a pudendal nerve, it may be desirable to locally fix distal end 48B of lead body 48.

In addition to including hydrogel fixation members located proximally to electrodes 50 of a lead, as shown in FIGS. 2-3, and located distally to electrodes 50, as shown in FIG. 5, a lead may include hydrogel fixation members located both proximally and distally to the electrodes carried by the lead, as shown in FIG. 6. FIG. 6 is a perspective view of lead 78, which includes two sets 80 and 82 of hydrogel fixation members. First set 80 includes hydrogel fixation members 52, 54, 56, and 60 (FIGS. 2-4B), while second set 82 includes hydrogel fixation members 72, 74, and 76 (FIG 5). First set 80 is located between electrodes 50 (adjacent to proximal electrode 50A) and proximal end 48A of lead body 48. Second set 82 is located between electrodes 50 (adjacent to distal electrode 50D) and distal end 48B of lead body 48. Fixing lead 78 both distal and proximal to electrodes 50 may locally fix electrodes 50, which may useful in applications where a clinician aims to implant lead 78 such that electrodes 50 are centered at a target stimulation site.

In another embodiment of a lead in accordance with the invention, hydrogel fixation members may also be disposed between electrodes 50 carried by a lead. For example, as shown in FIG. 7, lead 90 includes sets 80 and 82 of hydrogel fixation members, which were shown in FIG. 6, as well as set 91 of hydrogel fixation members. Sets 80, 82, and 91 are axially displaced from each other along lead body 48. As described above in reference to FIG. 6, first set 80 of hydrogel fixation members, which includes hydrogel fixation members 52, 54, and 56, is disposed between proximal electrode 50A and proximal end 48A of lead body 48. Second set 82 of hydrogel fixation members, which includes hydrogel fixation members 72, 74, and 76, is disposed between distal electrode 50D and distal end 48B of lead body 48. Third set 91 of hydrogel fixation members, which includes hydrogel fixation members 92, 94, and 96 disposed in recesses 93, 95, and 97, respectively, is disposed between electrodes 50B and 50C.

As depicted in FIG. 7, hydrogel fixation members 92, 94, and 96 have a smaller profile than hydrogel fixation members 52, 54, and 56. As a result, recesses 93, 95, and 97 may be smaller in dimension than recesses 53, 55, and 57, which may help minimize a diameter of lead 90 during implantation in a patient because an insulation layer of lead 90 may be thinner (i.e., a thickness of the insulation layer measured in a radial direction) to accommodate smaller recesses 93, 95, and 97. The smaller profile hydrogel fixation members 92, 94, and 96 may be attributable to many factors. For example, hydrogel fixation members 92, 94, and 96 may each be formed of a hydrogel material that is capable of greater expansion than hydrogel fixation members 52, 54, 56, and 60 of first set 80. Alternatively, hydrogel fixation members 92, 94, and 96 may each be formed such that hydrogel fixation members 92, 94, and 96 do not expand to as great of a dimension as hydrogel fixation members 52, 54, and 56. In other embodiments, hydrogel fixation members 92, 94, and 96 may have the relative profile size as hydrogel fixation members 52, 54, and 56.

In alternate embodiments, lead 90 may include hydrogel fixation members disposed between other electrodes 50. For example, hydrogel fixation members may be disposed between electrodes 50A and 50B and/or between electrodes 50C and 50D. In addition, first and/or second sets 80 and 82 of hydrogel fixation members may be removed from lead 90 in other embodiments. In yet other alternate embodiments, more than one set of hydrogel fixation members may be disposed between electrodes 50 and proximal end 48A of lead body 48, between electrodes 50 and distal end 48B of lead body 48, and between individual electrodes 50.

In the embodiment shown in FIG. 7, each set 80, 82, and 91 of fixation members includes the same number and same radial arrangement of hydrogel fixation members. For example, hydrogel fixation member 106 of set 82 has substantially the same radial position along lead body 48 as hydrogel fixation member 92 of set 91. In alternate embodiments, as shown in FIG 8, a lead may include more than two sets of hydrogel fixation members, where at least one fixation member from each set has a different radial position than a corresponding a fixation member in another other set. In addition, at least two of the sets (if the lead includes more than two sets) may include a different number of fixation members.

FIG. 8 shows a perspective view of lead 100, which includes sets 91, 102, and 104 of hydrogel fixation members. Set 91 of hydrogel fixation members is similar to set 91 of FIG. 7. Set 102 includes hydrogel fixation members 106 and 108, which are disposed in recesses 107 and 109, respectively, defined by lead body 48. Set 104 includes hydrogel fixation members 110 and 112, which are disposed in recesses 111 and 113, respectively, defined by lead body 48. Lead 100 is similar to lead 90 of FIG. 7 to the extent that both leads 90 and 100 include three sets of hydrogel fixation members. However, as discussed above, each set 80, 82, and 91 of hydrogel fixation members of lead 90 of FIG. 7 include fixation members that are at the same radial location. In contrast, in lead 100 of FIG. 8, hydrogel fixation members 106 and 108 of set 102 are at different radial positions than hydrogel fixation members 92, 94, and 96 of set 91. Hydrogel fixation members 106 and 108 of set 102, however, have substantially the same radial positions as hydrogel fixation members 110 and 112, respectively, of set 104, which is adjacent to proximal end 48B of lead body 48.

In other embodiments, other arrangements of the hydrogel fixation members of each set 91, 102, and 104 are contemplated. For example, in one embodiment, hydrogel fixation members 106 and 108 of set 102 may have different radial positions than hydrogel fixation members 110 and 112, respectively, of set 104.

FIG 9 is a perspective view of lead 120, which includes hydrogel fixation members 122 and 124 extending in different directions. More specifically, fixation member 122 extends toward proximal end 48A of lead body 48, while fixation member 124 extends toward distal end 48B of lead body 48. Hydrogel fixation members 122 and 124 sit in different positions within recesses 123 and 125, respectively, in order to accommodate the different directions of extension of the respective hydrogel fixation member 122 and 124 upon expansion to the second state. While both recesses 123 and 124 have width W, hydrogel fixation member 122 is seated closer to a distal side 123B of recess 123 than proximal side 123A. Similarly, hydrogel fixation member 124 is seated closer to a proximal side 125A of recess 125 than distal side 125B. Each of hydrogel fixation members 122 and 124 may or may not extend from lead body 48 at the same angle with respect to outer surface 48C of lead body 48.

While tine-shaped hydrogel fixation members are shown in FIGS. 2-9 above, a hydrogel fixation member may have any suitable shape. In addition, the hydrogel fixation member may also extend radially outward, without an angular component from lead body 48. where an "angular component" generally refers to an angle (e.g., angle J of FIG. 2) of greater than or less than about 90°. FIGS. 10A and 10B are a perspective view of lead 130 and schematic cross-sectional view of lead 130, respectively, which includes hydrogel fixation member 132 that extends around an outer perimeter of lead body 48. In FIG. 10A, hydrogel fixation member 132 is shown in a hydrated state (shown in phantom lines and indicated to be hydrogel fixation member 132'), in which hydrogel fixation member 132 protrudes past outer surface 48C of lead body 48 to engage with surrounding tissue. In an unexpanded state (FIG 10B), hydrogel fixation member 132 is disposed at least partially in recess 133 (FIG. 10B) defined by lead body 48. Recess 133 also extends around an outer perimeter of lead body 48 in order to accommodate hydrogel fixation member 132.

In the embodiment shown in FIGS. 10A and 10B, a single hydrogel fixation member 132 is disposed between electrodes 50 and proximal end 48A of lead body 48. In alternate embodiments, lead 130 may include any suitable number of hydrogel fixation members that extend around an outer perimeter of lead body 48 and/or hydrogel fixation member 132 may be used in combination with tine-like or other hydrogel fixation members that do not extend around the entire outer perimeter of lead body 48. For example, in another embodiment, hydrogel fixation member 132 may extend around 25 percent (%), 50% or 75% of the outer perimeter of lead body 48. Furthermore, although hydrogel fixation member 132 is shown in FIGS. 10A and 10B as expanding radially outward without an angular component, in alternate embodiments, hydrogel fixation member 132 may extend from lead body 48 at an angle with respect to outer surface 48C.

It is also noted that in other embodiments of leads in accordance with the invention, any one of the hydrogel fixation members shown in FIGS. 2-9 may also extend radially outward without an angular component, as shown in FIG 11 with respect to hydrogel fixation members 140 and 142 of lead 144. When hydrogel fixation members 140 and 142 expand radially outward at approximately 90° with respect to outer surface 48C of lead body 48, it may be possible for lead body 48 to define recesses 141 and 143 that are relatively smaller than recesses for angular hydrogel fixation members because recesses 141 and 143 do not have to accommodate angular extension of hydrogel fixation members 140 and 142, respectively. In an expanded state, as shown in phantom lines and indicated as hydrogel fixation members 140' and 142', hydrogel fixation members have a curvilinear cross-sectional shape.

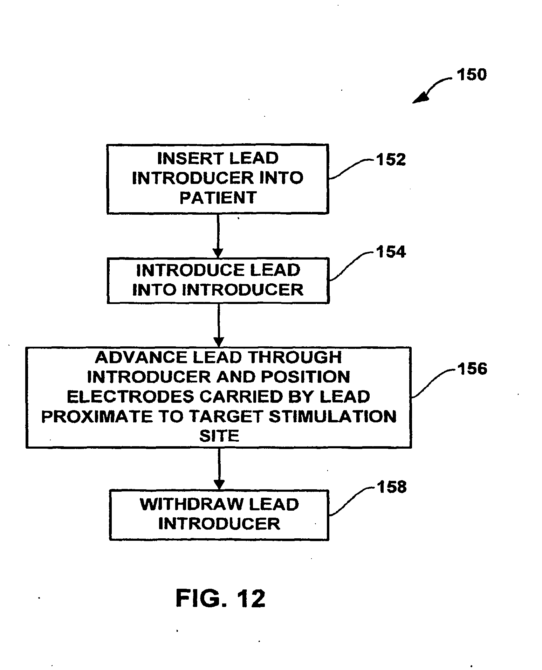

FIG. 12 is a flow diagram illustrating process 150 for implanting a lead 14 (FIGS. 1-3) including hydrogel fixation members in accordance with the invention. While lead 14 is referenced in the description of FIG. 12, it should be understood that process 150 may be used to implant any of leads 70, 78, 90, 100, 120, 130 or 144 of FIGS. 5-11, respectively, or any other lead including hydrogel fixation members in accordance with the invention.

A lead introducer, such as an introducer needle, is introduced into tissue of patient 16 (FIGS. 1A and 2) and a distal end of the introducer is guided to target stimulation site 18 (152). The introducer needle may be inserted into the patient percutaneously or via an incision (e.g., incision 33 in FIG. 1B). In one embodiment, the introducer is guided into a subcutaneous region of patient 16 in order to reach target stimulation site 18. Lead 14 is introduced into a lumen of the introducer (154). In particular, distal end 14B of lead 14 is introduced into the lumen before proximal end 14A.

Lead 14 is advanced through the lumen until electrodes 50 adjacent to distal end 48B of lead body 48B of lead 14 are positioned proximate to target stimulation site 18 (156). Positioning of the introducer and/or lead 14 may be aided by imaging techniques, such as by fluoroscopy using markers (e.g. radio-opaque or otherwise visible) on lead body 48. The markers may help indicate a location of hydrogel fixation members 52, 54, 56, and 60 with respect to the introducer needle. Distal end 14B of lead 14 is advanced through the lumen of the introducer until at least distal end 14B protrudes past the lumen and into tissue of patient 16 and hydrogel fixation members 52, 54, 56, and 60 are deployed from the introducer (i.e., are advanced past a distal end of the introducer). Alternatively, hydrogel fixation members 52, 54, 56, and 60 may be deployed from the introducer by withdrawing the introducer or another sheath separating hydrogel fixation members 52, 54, 56, and 60 from fluid, from patient 16, thereby exposing lead 14.

Upon deployment into body tissue, hydrogel fixation members 52, 54, 56, and 60 begin to absorb fluid from surrounding body tissue to expand into a hydrated state (i.e., hydrogel fixation members 52', 54', 56', and 60' shown in FIG. 4A). In the expanded state hydrogel fixation members 52, 54, 56, and 60 protrude out of recesses 53, 55, 57, and 61, respectively, and extend from lead body 48 to engage with surrounding tissue to substantially fix electrodes 50 proximate to stimulation target site 18. After lead 14 is positioned, the lead introducer is withdrawn from patient 16 (158).

A lead including expandable fixation members disposed in recesses in a lead body may be useful for various electrical stimulation systems. For example, in other embodiments, the lead may be used to deliver electrical stimulation therapy to patients to treat a variety of symptoms or conditions such as chronic pain, tremor, Parkinson's disease, multiple sclerosis, spinal cord injury, cerebral palsy, amyotrophic lateral sclerosis, dystonia, torticollis, epilepsy, pelvic floor disorders, gastroparesis, muscle stimulation (e.g., functional electrical stimulation (FES) of muscles) or obesity. In addition, while a lead is described above, in other embodiments, the fixation member arrangement described herein may be used to fix a fluid delivery conduit, such as a drug deliver catheter, proximate to a target drug delivery site.

## Claims

1. An apparatus comprising:
an implantable elongated member defining at least a first recess (93, 95, 97) and a second recess (73, 75, 77), and comprising a lead (14, 90, 100) including a lead body (48) extending between a proximal end (48A) and a distal end (48B), the elongated member being configured to couple to a medical device (12) to deliver a therapy from the medical device to a target therapy delivery site (18) in a patient, wherein the elongated member comprises a plurality of electrodes (50) disposed on the lead body proximate to the distal end of the lead body and at least one of the electrodes is positioned between the first and second recesses; **characterized by**
a first expandable fixation member (92, 94, 96) disposed in the first recess and configured to expand from a first dimension in a first state to a second dimension in a second state, wherein the first expandable fixation member is located between the at least one of the electrodes and the proximal end of the lead body; and
a second expandable fixation member (72, 74, 76) disposed in the second recess and located between the at least one of the electrodes and the distal end of the lead body.

2. The apparatus of claim 1, wherein the first expandable fixation member (92, 94, 96) is a hydrogel fixation member configured to expand from the first dimension in a substantially dehydrated state to the second dimension in a substantially hydrated state, or wherein the first expandable fixation member (92, 94, 96) is a shape memory fixation member.

3. The apparatus of claim 1, wherein the first expandable fixation member (92, 94, 96) is angled toward a proximal end of the elongated member and the second expandable fixation member (72, 74, 76, 124) is angled toward a distal end of the elongated member.

4. The apparatus of claim 1, wherein the first expandable fixation member (92, 94, 96) is at a first location along an outer perimeter of the elongated member and the second expandable fixation member (72, 74, 76) is at a second location along the outer perimeter of the elongated member, the second location being substantially opposite the first location.

5. The apparatus of claim 1, wherein in the second state, the first expandable fixation member (92, 94, 96) extends from the elongated member at an angle of less than 90° with respect to an outer surface of the elongated member, and preferably wherein in the second state, the first expandable fixation member (92, 94, 96) is angled toward a proximal end of the elongated member.

6. The apparatus of claim 1, wherein the first expandable fixation member (52, 92, 94, 96) is configured to break away from the elongated member.

7. The apparatus of claim 1, wherein in the second state, the first expandable fixation member (92, 94, 96) has a cross-sectional shape selected from a group consisting of: a rectangular shape and a curvilinear shape.

8. The apparatus of claim 1, wherein the first expandable fixation member (92, 94, 96) defines at least one of a tine or a flange-like structure when expanded to the second dimension.

9. The apparatus of claim 1, wherein the first recess (93, 95, 97, 133) extends around an outer perimeter of the elongated member.

10. The apparatus of claim 1, wherein an insulation layer (64) of the elongated member (14, 48, 90, 100) defines the first recess (93, 95, 97) and the second recess (73, 75, 77).

11. The apparatus of claim 1, wherein in the second state, the first expandable fixation member (92, 94, 96, 132, 140) extends radially outward from the elongated member (14, 48, 90, 100) at 90° with respect to an outer surface (48C) of the elongated member.

12. The apparatus of claim 1, wherein the first expandable fixation member (92, 94, 96) is attached to the elongated member (14, 48, 90, 100) with at least one of an adhesive (66) or an expandable sleeve (68).

13. The apparatus of claim 1, wherein the first expandable fixation member (92, 94, 96) is disposed between two electrodes (50) of the plurality of electrodes.

14. The apparatus of claim 1, wherein the elongated member comprises a catheter to deliver a fluid from the medical device to the target therapy delivery site.

15. A system (10) comprising:
a medical device (12); and
an apparatus (14, 70, 78, 90, 100, 120, 130, 144) of any of claims 1-14.

## Patentansprüche

1. Vorrichtung mit:
einem implantierbaren länglichen Element, das wenigstens eine erste Aussparung (93, 95, 97) und eine zweite Aussparung (73, 75, 77) definiert und eine Leitung (14, 90, 100) aufweist, die einen Leitungskörper (48) aufweist, der zwischen einem proximalen Ende (48A) und einem distalen Ende (48B) verläuft, wobei das längliche Element konfiguriert ist, um an eine medizinische Vorrichtung (12) gekoppelt zu werden, um eine Therapie von der medizinische Vorrichtung an eine Ziel-Therapieabgabestelle (18) in einem Patienten abzugeben, wobei das längliche Element mehrere Elektroden (50) aufweist, die an dem Leitungskörper unmittelbar am distalen Ende des Leitungskörpers angeordnet sind, wobei wenigstens eine der Elektroden zwischen den ersten und zweiten Aussparungen positioniert ist; **gekennzeichnet durch**
ein erstes expandierbares Befestigungselement (92, 94, 96), das in der ersten Aussparung angeordnet ist und konfiguriert ist, um aus einer ersten Größe in einem ersten Zustand zu einer zweiten Größe in einem zweiten Zustand zu expandieren, wobei sich das erste expandierbare Befestigungselement zwischen der wenigstens einen der Elektroden und dem proximalen Ende des Leitungskörpers befindet; und
ein zweites expandierbares Befestigungselement (72, 74, 76), das in der zweiten Aussparung angeordnet ist und sich zwischen der wenigstens einen der Elektroden und dem distalen Ende des Leitungskörpers befindet.

2. Vorrichtung nach Anspruch 1, wobei das erste expandierbare Befestigungselement (92, 94, 96) ein Hydrogel-Befestigungselement ist, das konfiguriert ist, um aus einer ersten Größe in einem im Wesentlichen dehydrierten Zustand zu einer zweiten Größe in einem im Wesentlichen hydratisierten Zustand zu expandieren, oder wobei das erste expandierbare Befestigungselement (92, 94, 94) ein Befestigungselement mit Formgedächtnis ist.

3. Vorrichtung nach Anspruch 1, wobei das erste expandierbare Befestigungselement (92, 94, 96) zu einem proximalen Ende des länglichen Elements abgewinkelt ist und das zweite expandierbare Befestigungselement (72, 74, 76, 124) zu einem distalen Ende des länglichen Elements abgewinkelt ist.

4. Vorrichtung nach Anspruch 1, wobei sich das erste expandierbare Befestigungselement (92, 94, 96) an einem ersten Ort längs eines äußeren Umfangs des länglichen Elements befindet und sich das zweite expandierbare Befestigungselement (72, 74, 76) an einem zweiten Ort längs des äußeren Umfangs des länglichen Elements befindet, wobei der zweite Ort dem ersten Ort im Wesentlichen gegenüberliegt.

5. Vorrichtung nach Anspruch 1, wobei sich im zweiten Zustand das erste expandierbare Befestigungselement (92, 94, 96) in einem Winkel von weniger als 90° bezüglich einer Außenfläche des länglichen Elements vom länglichen Element erstreckt und bei dem das erste expandierbare Befestigungselement (92, 94, 96) im zweiten Zustand vorzugsweise zu einem proximalen Ende des länglichen Elements abgewinkelt ist.

6. Vorrichtung nach Anspruch 1, wobei das erste expandierbare Befestigungselement (52, 92, 94, 96) konfiguriert ist, um vom länglichen Element abzubrechen.

7. Vorrichtung nach Anspruch 1, wobei das erste expandierbare Befestigungselement (92, 94, 96) im zweiten Zustand eine Querschnittsform besitzt, die aus einer Gruppe
ausgewählt ist, die eine rechteckige Form und eine krummlinige Form umfasst.

8. Vorrichtung nach Anspruch 1, wobei das erste expandierbare Befestigungselement (92, 94, 96) eine Zacke und/oder eine flanschähnliche Struktur definiert, wenn es zu der zweiten Größe expandiert ist.

9. Vorrichtung nach Anspruch 1, wobei die erste Aussparung (93, 95, 97, 133) um einen äußeren Umfang des länglichen Elements verläuft.

10. Vorrichtung nach Anspruch 1, wobei eine Isolationsschicht (64) des länglichen Elements (14, 48, 90, 100) die erste Aussparung (93, 95, 97) und die zweite Aussparung (73, 75, 77) definiert.

11. Vorrichtung nach Anspruch 1, wobei sich das erste expandierbare Befestigungselement (92, 94, 96, 132, 140) im zweiten Zustand vom länglichen Element (14, 48, 90, 100) um 90° bezüglich einer Außenfläche (48C) des länglichen Elements radial nach außen erstreckt.

12. Vorrichtung nach Anspruch 1, wobei das erste expandierbare Befestigungselement (92, 94, 96) mit einem Klebstoff (66) und/oder einer expandierbaren Manschette (68) am länglichen Element (14, 48, 90, 100) befestigt ist.

13. Vorrichtung nach Anspruch 1, wobei das erste expandierbare Befestigungselement (92, 94, 96) zwischen zwei Elektroden (50) der mehreren Elektroden angeordnet ist.

14. Vorrichtung nach Anspruch 1, bei der das längliche Element einen Katheter aufweist, um ein Fluid von der medizinischen Vorrichtung an die Ziel-Therapieabgabestelle abzugeben.

15. System (10), das enthält:
eine medizinische Vorrichtung (12); und
eine Vorrichtung (14, 70, 78, 90, 100, 120, 130, 144) nach einem der Ansprüche 1-14.

## Revendications

1. Dispositif comportant :
un élément allongé implantable définissant au moins un premier évidement (93, 95, 97) et un second évidement (73, 75, 77), et comportant une dérivation (14, 90, 100) incluant un corps de dérivation (48) s'étendant entre une extrémité proximale (48A) et une extrémité distale (48B), l'élément allongé étant configuré pour être couplé à un dispositif médical (12) afin d'administrer une thérapie depuis le dispositif médical à un site d'administration de thérapie cible (18) dans un patient, dans lequel l'élément allongé comporte une pluralité d'électrodes (50) disposées sur le corps de dérivation à proximité de l'extrémité distale du corps de dérivation et au moins l'une des électrodes est positionnée entre les premier et second évidements ; **caractérisé par**
un premier élément de fixation extensible (92, 94, 96) disposé dans le premier évidement et configuré pour s'étendre depuis une première dimension dans un premier état vers une seconde dimension dans un second état, dans lequel le premier élément de fixation extensible est localisé entre la au moins une des électrodes et l'extrémité proximale du corps de dérivation, et
un second élément de fixation extensible (72, 74, 76) disposé dans le second évidement et localisé entre la au moins des électrodes et l'extrémité distale du corps de dérivation.

2. Dispositif selon la revendication 1, dans lequel le premier élément de fixation extensible (92, 94, 96) est un élément de fixation hydrogel configuré pour s'étendre depuis la première dimension dans un état en grande partie déshydraté vers la seconde dimension dans un état en grande partie hydraté, ou dans lequel le premier élément de fixation extensible (92, 94, 96) est un élément de fixation à mémoire de forme.

3. Dispositif selon la revendication 1, dans lequel le premier élément de fixation extensible (92, 94, 96) est orienté vers une extrémité proximale de l'élément allongé et le second élément de fixation extensible (72, 74, 76, 124) est orienté vers une extrémité distale de l'élément allongé.

4. Dispositif selon la revendication 1, dans lequel le premier élément de fixation extensible (92, 94, 96) est à un premier remplacement le long d'un périmètre extérieur de l'élément allongé et le second élément de fixation extensible (72, 74, 76) est à un second emplacement le long du périmètre extérieur de l'élément allongé, le second emplacement étant sensiblement opposé au premier remplacement.

5. Dispositif selon la revendication 1, dans lequel dans un second état, le premier élément de fixation extensible (92, 94, 96) s'étend depuis l'élément allongé selon un angle inférieur à 90° par rapport à une surface extérieure de l'élément allongé et, de manière préférée, dans lequel dans le second état, le premier élément de fixation extensible (92, 94, 96) est orienté vers une extrémité proximale de l'élément allongé.

6. Dispositif selon la revendication 1, dans lequel le premier élément de fixation extensible (52, 92, 94, 96) est configuré pour se séparer de l'élément allongé.

7. Dispositif selon la revendication 1, dans lequel dans le second état, le premier élément de fixation extensible (92, 94, 96) a une forme en coupe sélectionnée à partir d'un groupe constitué de : une forme rectangulaire et une forme curvilinéaire.

8. Dispositif selon la revendication 1, dans lequel le premier élément de fixation extensible (92, 94,96) définit au moins l'une d'une structure de type bride ou dent lorsque étendu dans la seconde dimension.

9. Dispositif selon la revendication 1, dans lequel le premier évidement (93, 95, 97, 133) s'étend autour d'un périmètre extérieur de l'élément allongé.

10. Dispositif selon la revendication 1, dans lequel une couche isolante (104) de l'élément allongé (14, 48, 90, 100) définit le premier évidement (93, 95, 97) et le second évidement (73, 75, 77).

11. Dispositif selon la revendication 1, dans lequel dans le second état, le premier élément de fixation extensible (92, 94, 96, 132, 140) s'étend radicalement vers l'extérieur à partir de l'élément allongé (14, 48, 90, 100) à 90° par rapport à une surface extérieure (48C) de l'élément allongé.

12. Dispositif selon la revendication 1, dans lequel le premier élément de fixation extensible (92, 94, 96) est fixé à l'élément allongé (14, 48, 90, 100) à l'aide d'au moins un adhésif (66) ou un manchon extensible (68).

13. Dispositif selon la revendication 1, dans lequel le premier élément de fixation extensible (92, 94, 96) est disposé entre deux électrodes (50) parmi la pluralité d'électrodes.

14. Dispositif selon la revendication 1, dans lequel l'élément allongé comporte un cathéter pour administrer un fluide à partir du dispositif médical vers le site d'administration de thérapie cible.

15. Système (10) comportant :
un dispositif médical (12) ; et
un dispositif (14, 70, 78, 90, 100, 120, 130, 144) selon l'une quelconque des revendications 1 à 14.
